# EUROPÄISCHE PATENTSCHRIFT

(11) **EP 0 390 829 B1**
(45) Veröffentlichungstag und Bekanntmachung des Hinweises auf die Patenterteilung: **17.03.1993**
(21) Anmeldenummer: 89900229.9
(22) Anmeldetag: 07.12.1988
(51) Int. Cl.: C12N 5/00, C12N 5/02

(54) **VERFAHREN ZUR ERZEUGUNG SYNGENER ZELLEN UND DEREN VERWENDUNG**
PROCESS FOR PRODUCING SYNGENEIC CELLS, AND THEIR USE
PROCEDE DE PRODUCTION DE CELLULES SYNGENETIQUES

(30) Priorität: 09.12.1987 DE 3741606
(43) Veröffentlichungstag der Anmeldung: 10.10.1990
(73) Patentinhaber: Stahl, Kurt-Wilhelm, Prof. Dr. Dr., D-79100 Freiburg (DE)
(72) Erfinder: Stahl, Kurt-Wilhelm, Prof. Dr. Dr., D-79100 Freiburg (DE)
(74) Vertreter: Türk, Gille, Hrabal, Leifert
(86) Internationale Anmeldenummer: EP8801118
(87) Internationale Veröffentlichungsnummer: WO8905343

(56) Entgegenhaltungen:
- The European Journal of Immunology, vol. 9, 1979, Verlag Chemie GmbH (Weinheim, DE) Y.H. Chang et al "Investigation of the human macrophage I. Collection and in vitro cultivation", pages 517-525
- The New England Journal of Medicine, vol. 313, n 23, 3 December 1985 S.A. Rosenberg et al "Observations on the systemic administration of autologous lymphokine-activated killer cells and recombinant interleukin-2 to patients with metastatic cancer", pages 1485-1492
- Clinical Research, vol. 35, n 3, 1987, page 681A, K.M. Ellner et al "Synergism between triphosphoron azegelii and coryneform bacteria from genital white fiedra"
- Clinical Research, vol. 31, n 2, 1983, page 264, E.W.B. Jeffes et al "Lymphocyte derived cytolytic molecules (Lymphotoxin) are present in the blister fluid of bullous penphigold patients"

## Beschreibung

Die Erfindung betrifft ein Verfahren zur Erzeugung syngener Zellen (Zellen mit identischer Erbanlage) in vivo in einer Blase zwischen Dermis und Epidermis oder innerhalb der Epidermis von Mensch oder Tier. Sie betrifft auch die Verwendung der erhaltenen syngenen Zellen für biotechnische sowie therapeutische Zwecke und zur Bereitung und/oder Testung von Arzneimitteln.

Es ist bekannt, daß in die Flüssigkeit von Blasen, die zwischen Dermis und Epidermis oder innerhalb der Epidermis durch chemische oder mechanische Einwirkung gebildet werden, gewisse Körperzellen (Entzündungszellen) einströmen. Hautblasen wurden daher aus klinischen und experimentellen Gründen erzeugt. Klinische Gründe lagen in der Möglichkeit, Entzündungsreaktionen 24 bis 48 Stunden nach der Induktion der Blase quantitativ zu bewerten. Man hoffte, bei Patienten mit schweren Erkrankungen, wie Krebserkrankungen, eine Korrelation zwischen der Menge der in der Blasenflüssigkeit gefundenen weißen Blutkörperchen und dem Immunitätszustand des Patienten und damit dem Zustand der Erkrankung zu finden. Experimentell wurde versucht, Makrophagen zu gewinnen, die von Monocyten in dem peripheren Blut stammen, von wo sie chemotaktisch angelockt in das Gewebe einwandern, wo sie zu Makrophagen ausreifen. Es ist jedoch sehr schwierig, bei gesunden Donoren ausreichende Mengen an Makrophagen in einem zufriedenstellenden Reinheitsgrad zu erzielen (Yu-hui Chang and Chin-sheng Yao in The European Journal of Immunology 1979, 9, 517-525: "Investigation of the human macrophage 1: Collection and in vitro cultivation, 2: the in vitro cytotoxicity of macrophage").

Durch Blasenbildung mittels Cantharidin konnte eine gewisse Anzahl menschlicher Makrophagen aus dem Blasenexsudat nach 48 Stunden gewonnen werden. Diese Zellen konnten zur Proliferation in vitro gebracht werden und zeigten die Morphologie und funktionellen Charakteristika von Makrophagen, was bedeutet, daß sie sich auch in vitro gegen Tumorzellen als zytotoxisch erwiesen. Die nach 48 Stunden erhaltene Blasenflüssigkeit wurde auch für therapeutische Zwecke an zwei Krebspatienten untersucht, denen das autologe Blasenfluid topisch in die Tumoren injiziert wurde. Dabei wurde eine partielle Regression festgestellt.

Durch Unterdruck erzeugte Blasen wurden ausschließlich für experimentelle Zwecke verwendet, nämlich als Methode zur Gewinnung weißer Blutkörperchen, um deren Verhalten unter Arzneimitteleinwirkung zu untersuchen ("A simple method for studying chemotaxis, vascular pereability and histological modifications induced by mediators of inflammation in vivo in man", L. Michel, L. Dubertret, BJD (1985) 113, Suppl. 28, 61-66).

Eine Transfer-Therapie bei Krebspatienten führte zu Teilerfolgen. Es wurden Lymphozyten aus dem peripheren Blut von Krebspatienten isoliert und in vitro mit dem rekombinanten lnterleukin-2 Lymphokin- und Lymphozyten-Wachstumsfaktor des Menschen behandelt (rIL-II) ("Observations on the systemic administration of autologous lymphokine-activated killer cells and recombinant interleukin-2 to patients with metastatic cancer" A. Steven et al. The New England Journal of Medicine, Vol. 313, 23, 1485-1492 (1985)).

Die Lymphozyten wurden ausschließlich in vitro mit rIL-II behandelt: Die Zellen sollten mit Hilfe des Wachstumsfaktors rIL-II zur Proliferation angeregt werden; außerdem sollten sie in sogenannte Lymphokinaktivierte Killerzellen (LAK-Zellen), die für Tumoren zytotoxisch sind, umgewandelt werden. Es hat sich gezeigt, daß hohe Dosen an rIL-II zu unerwünschten Nebenwirkungen führen, jedoch hat es sich gezeigt, daß die autologe Zellbehandlung verschiedener Erkrankungen, wie Krebserkrankungen, wirksam mittels einer nicht-chemischen Therapie behandelt werden kann, vorausgesetzt, daß die dem Spender zurückinjizierten Zellen in ausreichender Anzahl zur Verfügung stehen. Dabei hat es sich jedoch erwiesen, daß die in vitro-Zellkultur verschiedene Nachteile aufweist. So benötigen die Zellen eine lange Anpassungszeit, um in vitro zu proliferieren. Es besteht die Gefahr einer bakteriellen oder viralen Infektion der in vitro-Kultur, und bei der Wiederverwendung der in vitro gewonnenen Zellen beim Donor muß eine neue Anpassungs-Periode durchlaufen werden.

Aufgabe der Erfindung war daher die Bereitstellung eines Verfahrens zur Erzeugung syngener Zellen, das die vorstehenden Nachteile der in vitro-Vermehrung nicht aufweist und zu einer brauchbaren Menge an erwünschten syngenen Zellen führt.

Diese Aufgabe wird erfindungsgemäß durch ein Verfahren zur Erzeugung syngener Zellen in vivo in einer Blase zwischen Dermis und Epidermis oder innerhalb der Epidermis von Mensch oder Tier gelöst, welches dadurch gekennzeichnet ist, daß man eine Vermehrung und Konditionierung der Zellen durch Einführen eines oder mehrerer zellspezifischer Waschstumsfaktoren (Peptide und Proteine), die durch rezeptor vermittelte Endozytose aufgenommen werden und die Zellteilung (Mitose) und Differenzierung fördern, nicht toxischer Konzentrationen von einem oder mehreren Oxidationsmitteln, deren Redoxpotential dem des Peroxidase-Wasserstoffperoxid-Komplexes entspricht oder einer Kombination beider, in die Blase bewirkt und daß die Zellen in steriler Weise geerntet werden. Es hat sich nämlich gezeigt, daß auf diese Weise eine Vermehrung und Konditionierung der Zellen erzielt werden kann, ohne daß hierbei systemische Auswirkungen auf den Körper auftreten. Es wurde gefunden, daß nicht-toxische Konzentrationen von Oxidationsmitteln, deren Redoxpotential dem des natürlichen Peroxidase-Wasserstoffperoxid-Komplexes entspricht (siehe S.J. Klebanoff und H. Rosen in Oxygen free Radicals and Tissue Damage (Ciba Found. Symposium 65, New Series); Excerpta Medica, Amsterdam 1979, Seiten 263-284), eine mitogene Wirkung haben. Dies bedeutet, daß geeignete Oxidantien in der Lage sein sollten, Ethylen durch Oxidation aus 4-(Methylthio)-2-oxybuttersäure (KMB) oder aus dem Pflanzenhormon 1-Aminocyclopropan -Carbonsäure (ACC) herauszufragmentieren (R.J. Youngman et al, Zeitschrift für Naturforschung, Band 40 C, Seiten 409-414, 1985).

Aus Eur.J.Immunol. 1979, 9, 517-520 ist es bekannt, durch Einführen von Stimulanzien in eine Hautblase die Zellausbeute an menschlichen Makrophagen zu vermehren. Die dort erwähnten Substanzen rufen als Stimulation einen vermehrten Einstrom von Zellen in die Blase hervor. Wachstumsfaktoren rufen jedoch als Peptide eine Zellvermehrung durch Zellteilung hervor. Das erfindungsgemäße Verfahren führt daher zu einer grundsätzlich anderen Wirkung, die nicht vorhersehbar war. Im Rahmen der vorliegenden Erfindung hat es sich überraschenderweise gezeigt, daß im Gegensatz zur bisherigen Technik, bei der eine geringe Monocyten-Menge in die Blasenflüssigkeit einströmte, nunmehr eine Vermehrung der eingeströmten Monocyten innerhalb der Blase in vivo erzielt werden kann, wenn, wie vorstehend ausgeführt, zellspezifische Wachstumsfaktoren und/oder mitogene mikromolare Konzentrationen von Oxidationsmitteln, deren Redoxpotential dem des Peroxidase-Wasserstoffperoxid-Komplexes entspricht, in die Blase eingeführt werden und die Blase länger als 3 Tage, vorzugsweise mindestens 5 Tage belassen wird.

Erfindungsgemäß wird zuerst eine intradermale Blase, entweder zwischen Dermis und Epidermis oder innerhalb der Epidermis ausgebildet. Eine derartige Blase wird, wie in der Literatur beschrieben, beispielsweise durch Cantharidin-Injektion oder durch Unterdruck gebildet. Erfolgt die Blasenerzeugung durch Cantharidin-Injektion, so wird das Cantharidin zweckmäßig in einer Lösung, die mindestens 1 mmolar ist, in einem geeigneten Träger, vorzugsweise in Ethanol eingesetzt. Eine bevorzugte Cantharidin-Konzentration ist 2 bis 5 mmolar.

In die so erzeugten Blasen wandern Entzündungszellen wie Granulozyten, Monozyten, unter Ausreifung in Makrophagen und Lymphozyten ein. In den intradermalen Blasen sterben Keratinozyten ab und setzen dabei das Lymphokin und den Wachstumsfaktor Interleukin I (rIL-I) frei. Das stellt eine besonders günstige Ausgangssituation für die Vermehrung von weißen Blutkörperchen (z.B. Lymphozyten und Makrophagen) dar: das an rIL-I angereicherte Medium wirkt einerseits chemotaktisch auf periphere Blutleukozyten, andererseits begünstigt es die Produktion weiterer Wachstumsfaktoren durch diese Zellen.

Nunmehr wurde gefunden, und hierauf beruht die Erfindung, daß eine Vermehrung und Konditionierung derartiger Zellen in den Haut-Blasen in vivo durchgeführt werden kann. Hierzu ist es, wie oben erwähnt, erforderlich, entsprechende zellspezifische Wachstumsfaktoren und/oder Oxidationsmittel in die Blasenflüssigkeit einzuführen und die Blase mehr als 3 Tage zu belassen (z.B. bis zu 10 Tagen oder mehr). Die Einführung erfolgt im allgemeinen einmal täglich, sie ist jedoch auch in größeren Intervallen von beispielsweise 2 bis 3 Tagen möglich.

Wachstumsfaktoren im Sinne der Erfindung sind Peptide und Proteine, die durch rezeptorvermittelte Endozytose aufgenommen werden und die Zellteilung (Mitose) und Differenzierung fördern. Beispiele hierfür sind Interleukin II, Interferone, Thymosin alpha I und dessen Fragmente, GM-CSF (Granulocyte Makrophage Colony Stimulating Factor) und GCSF (Granulocyte Colony Stimulating Factor), NGF, EGF, PDGF, ILGF, FGF, Transferrin und Erythropoietin.

Beispiele für Oxidationsmittel gemäß obiger Definition sind Wasserstoffperoxid und dessen Derivate wie z.B. Benzoylperoxid bzw. Anlagerungsverbindungen, Hemin, Ubichinon, Tetrachlordekaoxigen-Anionen-Komplex (TCDO gemäß DE-OS 36 00 931) und Perjodat.

Das Einführen erfolgt beispielsweise durch Injektion, z.B. durch Injektion von Lösungen der Wachstumsfaktoren in (beispielsweise 50 µl) sterilem isotonischem Phosphatpuffer (PBS, pH 7,4) wenn Wachstumsfaktoren alleine, d.h. ohne zusätzliche Oxidantienzugabe verabreicht werden. In diesem Fall ist zur Erzielung des gleichen Effektes wie mit der kombinierten Oxidantiengabe infolge der Kurzlebigkeit der Peptidfaktoren eine mehrmalige (z.B. bis zu dreimal pro Tag) tägliche Verabreichung in die Blase günstig.

Es hat sich auch als günstig erwiesen, zunächst Oxidationsmittel einzuführen, und zwar in nicht-toxischer Konzentration (z.B. für Perjodat 5 µmol, für Wasserstoffperoxid 30 nmol und für TCDO 3 µmol, jeweils pro ml Blasenflüssigkeit) und anschließend z.B. eine Stunde später, mit zellspezifischen Wachstumsfaktoren nachzubehandeln. Infolge der Potenzierung von Wachstumsfaktoren reicht dann im allgemeinen eine einmalige Gabe pro Tag.

Es hat sich nämlich gezeigt, daß hierdurch eine Potenzierung der durch die Wachstumsfaktoren angeregten Effekte (Vermehrung und Konditionierung bzw. mitogene Wirkung und Chemotaxies) erfolgen kann.

Außer den essentiellen Zusätzen, also Wachstumsfaktoren oder/und Oxidationsmitteln, können zusätzlich in die Hautblase auch andere Wirkstoff, insbesondere Pharmaka, eingeführt werden.

Das erfindungsgemäße Verfahren hat sich insbesondere zur Vermehrung von Lymphözyten und Makrophagen, vermehrungsfähiger Knochenmarkszellen, sowie von den Zellen, deren Wachstum von Makrophagen und T-Lymphozyten kontrolliert wird (z.B. Fibroblasten, Endothelzellen, Epidermiszellen, Leberparenchymzellen) bewährt.

Die jeweils verwendeten Wachstumsfaktoren werden in Abhängigkeit vom Zellentyp gewählt, z.B. Interleukin II für Lymphozyten, GM-CSF für Makrophagen, PDGF für Fibroblasten und Endothelzellen, EGF für Epidermiszellen.

Es hat sich erfindungsgemäß gezeigt, daß die Vermehrung der Makrophagen auch durch alleinigen Einsatz von Oxidationsmitteln oder einem kombinierten Einsatz von Wachstumsfaktoren und Oxidationsmitteln der angegebenen Art bewirkt werden kann. Beispiele sind mikromolare nicht-toxische Konzentrationen von Wasserstoffperoxid, Hemin, anorganischen oder organischen Oxidationsmitteln, z.B. Ubichinon, Benzoylperoxid, Tetrachlordekaoxygen-Anionen-Komplex (TCDO, wie nach DE-OS 36 00 931 hergestellt), millimolare Konzentrationen von Perjodat oder jegliche andere Verbindung die geeignet ist, die Phagozytose zu fördern, die über die NADPH-Oxygenase zur Bildung von reduzierten Sauertoffspezies führt, die selbst mitogen sind, wie das Peroxidanion, sowie das OH-Radikal und die im Makrophagen selbst die Synthese eines die Monozyten vermehrenden mitogenen Faktors anregen (FIM, 30 KDalton, Vortrag von Prof. Ralph von Furth, Universität Leyden, auf dem Symposium Immunologische Tage in Münster am 30. und 31. Oktober 1987). Derartige Oxidationsmittel wirken mitogen auf Makrophagen selbst, sie erhöhen jedoch auch die zelluläre Kooperation von Makrophagen mit Lymphozyten und anderen Zellen, wie Fibroblasten und Endothelzellen, wodurch eine verstärkte Proliferation dieser Zellen erfolgt. Ein Lösungsmittel für die Oxidationsmittel ist z.B. auch PBS, wie für die Wachstumsfaktoren beschrieben.

Erfindungsgemäß kann so vorgegangen werden, daß zunächst eine Blase erzeugt wird. Etwa 24 Stunden nach dem Setzen der Blase (Yu-hui Chang und Chin-sheng Yao, Loc.cit.; L. Michel et al, Loc.cit.) werden durch subkutanes Unterstechen am Rande der Blasen (die Blasenwand bleibt dabei intakt) etwa 50 bis 100 µl steriler Lösungen der einzuführenden Mittel mittels beispielsweise einer Tuberkulinspritze mit feiner Nadel eingeführt.

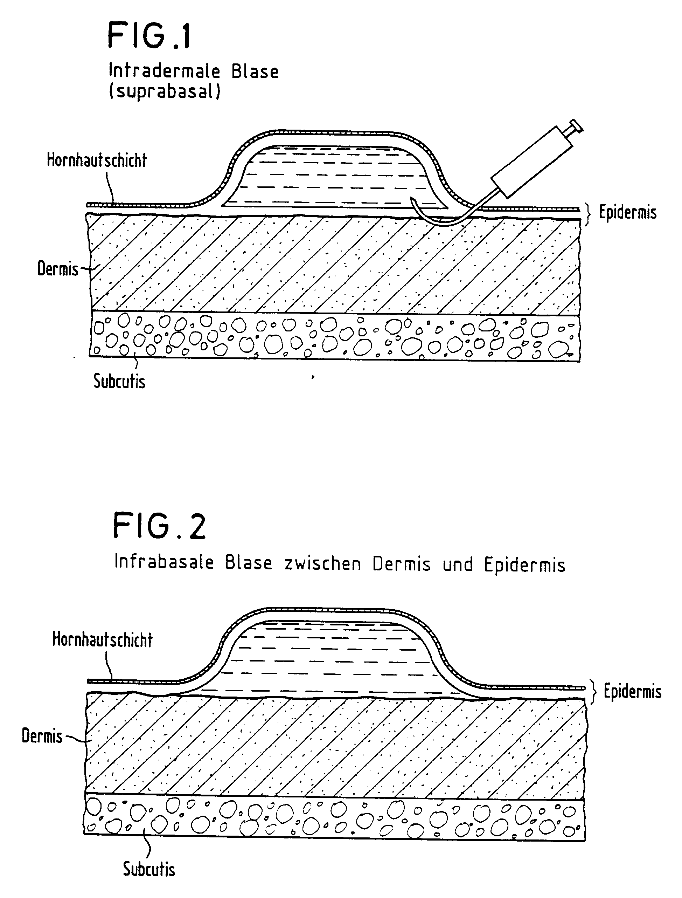

In der beigefügten Zeichnung veranschaulichen Figur 1 und 2 eine intradermale Blase, die wie vorstehend erläutert, unterstochen wird; bzw. eine infrabasale Blase zwischen Epidermis und Dermis. Wenn eine Vermehrung von Knochenmarkzellen, Fibroblasten, TIL-Zellen (Tumor Infiltrating Lymphocytes), Leber- und Hautzellen oder anderer Zellen erfolgen soll, so wird ein Teil der Blasenflüssigkeit entfernt und ersetzt durch ein steriles Kulturmedium mit einem Gehalt der zu vermehrenden Zellen, beispielsweise in einer Menge von etwa 10⁵ Zellen.

Die nach dem erfindungsgemäßen Verfahren vermehrten Zellen werden schließlich in steriler Weise gewonnen. Der Zugang zur Blase erfolgt analog zu dem Verfahren bei der intravesikalen Einspritzung. Nach Entleerung der Blase kann ein Spülvorgang mit sterilem Zellkulturmedium (beispielsweise etwa 1 ml) erfolgen, um die Ausbeute zu erhöhen.

Die Zellen können für biotechnologische Zwecke direkt verwendet werden; jedoch ist es auch möglich, sie gefroren zu konservieren. Zur biotechnologischen Weiterverarbeitung können die Zellen beispielsweise in vitro weiter kultiviert werden. Z.B. können Hybridomazellen in üblicher Weise in vitro zur Gewinnung monoklonaler Antikörper gezüchtet und die MAK gewonnen werden. Mit bestrahlten Tumorzellen können entsprechende körpereigene zytotoxische Lymphozyten erzeugt werden.

Durch die erfindungsgemäße Arbeitsweise wird es möglich, die Zellbildung, z.B. die Makrophagenbildung in vivo, 10-100fach heraufzusetzen. Die primäre Zellkultur erfolgt dabei in einem geeigneten Zellkulturmedium, das zunächst etwa 20 % der Blasenflüssigkeit enthält. Bei Erneuerung des Zellkulturmediums kann beispielsweise ein 5 bis 50 %iger Serumzusatz des Spenders benutzt werden.

Für therapeutische Zwecke können die in vivo kultivierten Zellen nach ihrer Gewinnung direkt an den Donor verabreicht werden; sie können jedoch auch zunächst in vitro (nach dem vorstehend beschriebenen Verfahren) noch weiter propagiert und erst anschließend an den Donor verabreicht werden. Die Verabreichung kann beispielsweise intravenös, intraperitoneal, subcutan, intramuskulär, intralesional oder intracerebral erfolgen. Auf diese Weise zuerst in vivo und danach in vitro vermehrte TIL-Zellen eignen sich z.B. als Antitumormittel (Cancer Research 48, 206-214; Immunology today 9, 58-61).

Antiidiotypische, in der Hautblase erzeugte B-Zellen und die daraus gewonnenen (monoklonalen) Antikörper sowie antiidiotypische T-Zellklone eignen sich in hervorragender Weise zur Behandlung von Autoimmunerkrankungen. Dafür werden patienteneigene Seren bzw. in vitro-vorstimulierte Zellen eingesetzt.

Biotechnisch können die hergestellten syngenen Zellen beispielsweise zur Produktion von Interferon durch permanente Makrophagenlinien verwendet werden. Auch ist die Benutzung etablierter Zellinien zur Austestung von neu zu entwickelnden Pharmaka (wodurch Tierversuche wegfallen können) oder zur Testung der Chemotherapieempfindlichkeit von normalen Zellen im Vergleich zu Tumorzellen möglich.

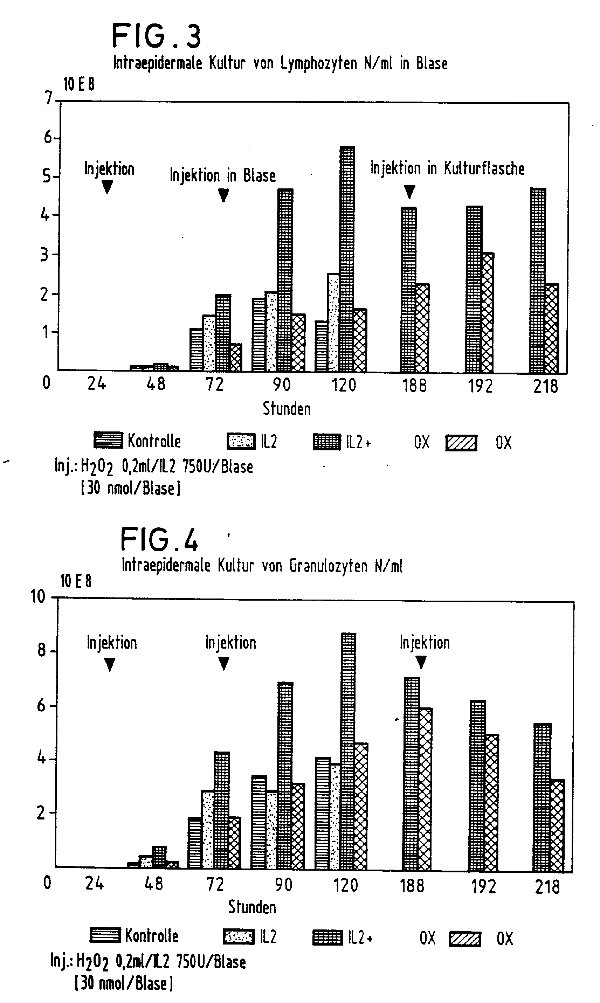

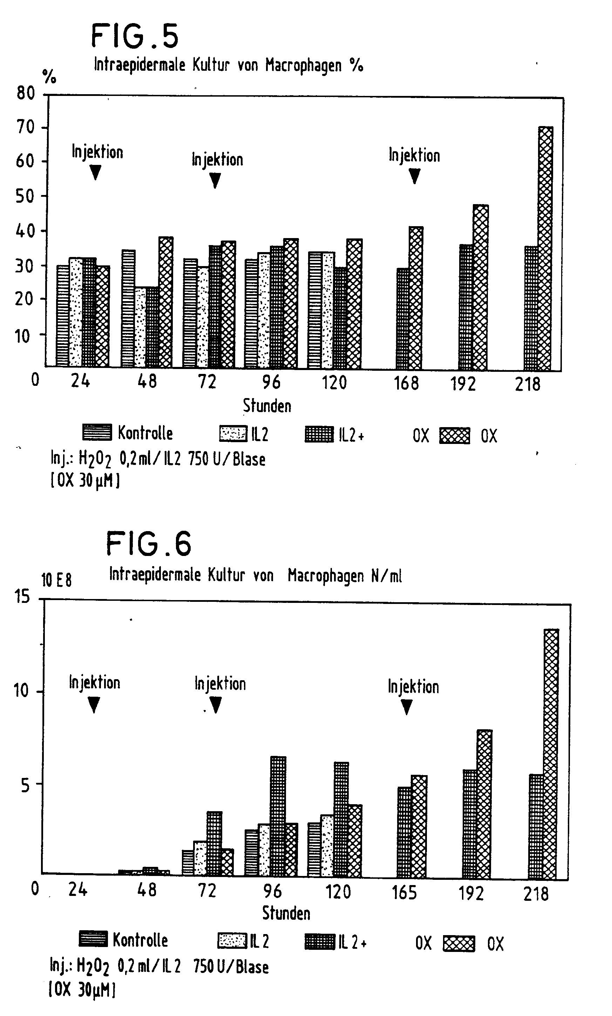

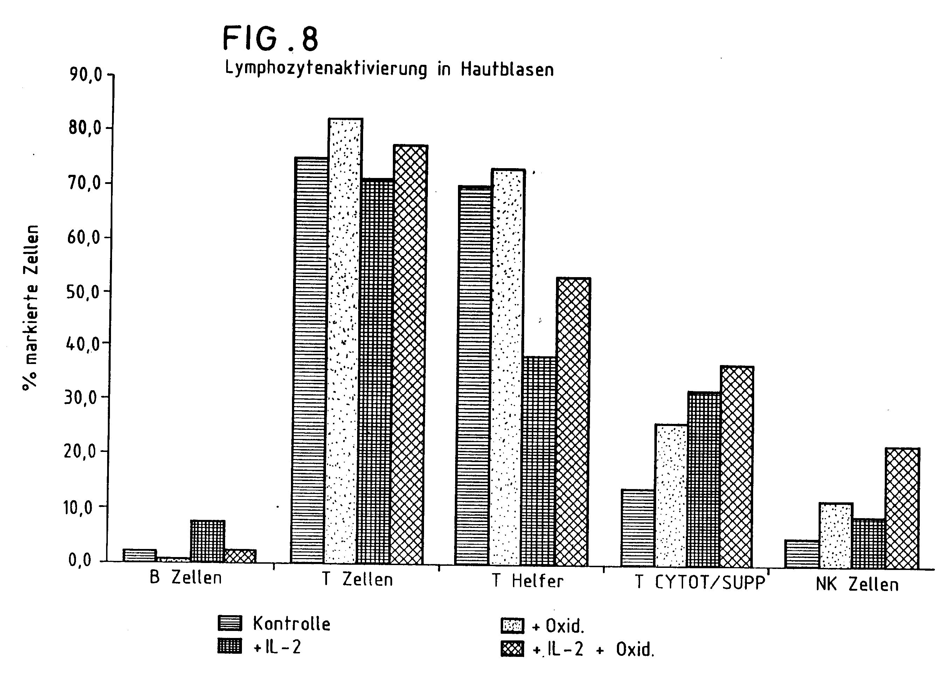

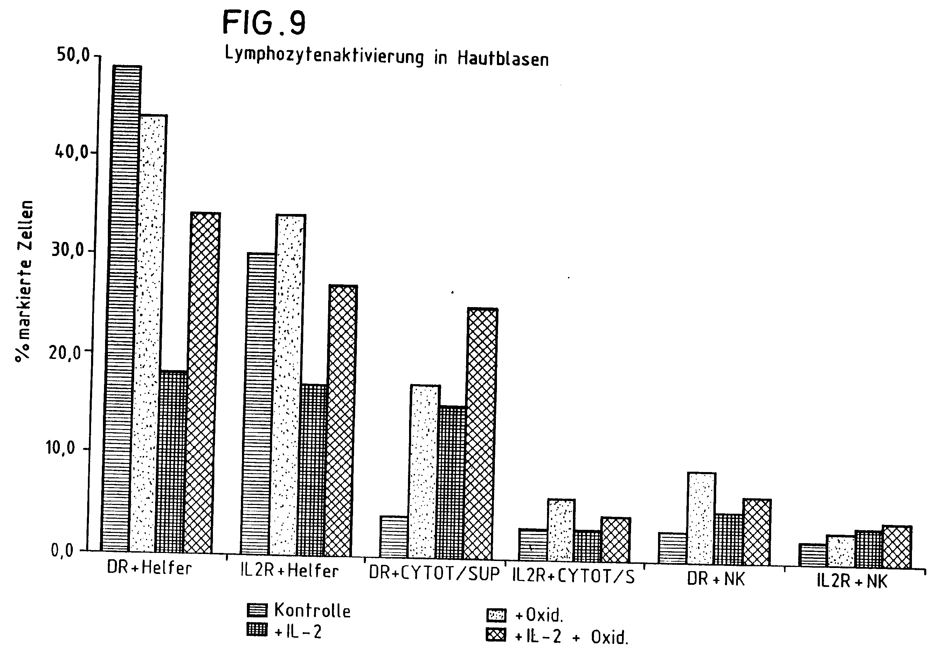

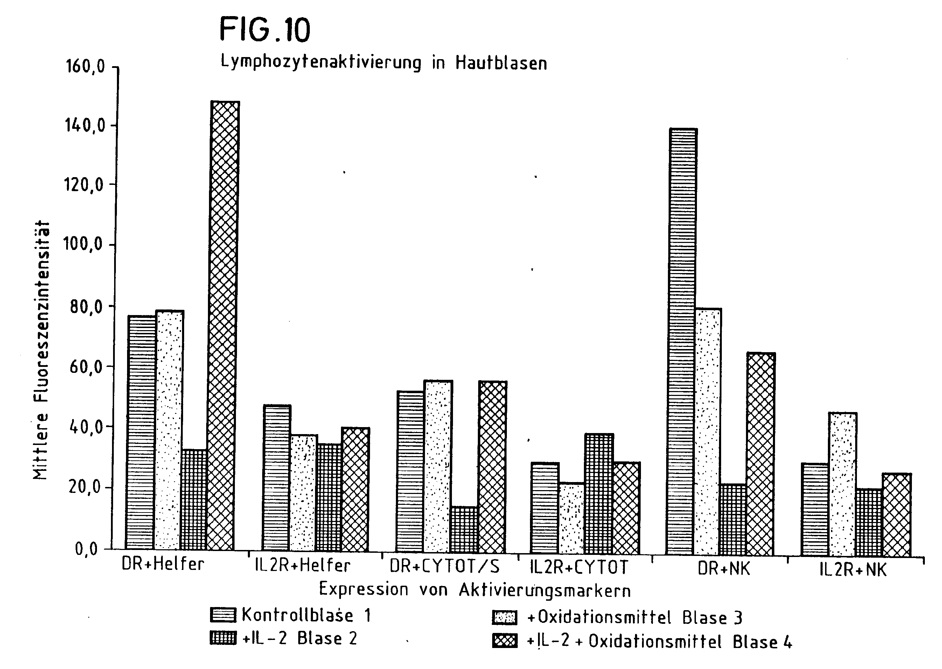

Die folgenden Beispiele erläutern die Erfindung in Verbindung mit den Figuren 3 bis 10 der Zeichnung weiter.

### Beispiel 1

Ein Okklusionsverband mit einem 1 x 1 cm großen Filterpapier mit in 70 % Ethanol 3 mmolarer Cantharidinlösung (Cantharidin Sigma Chemicals) wurde 5 Stunden belassen. Anschließend wird die entstehende Blase mit einem umgekehrten Petrikulturschälchen gegen mechanische Verletzungen geschützt. Intravesikale Applikationen (50 µl sterile PBS, 50 µl sterile PBS enthaltend 750 Units Interleukin II (rIL-II), 50 µl PBS enthaltend 30 µmol H₂O₂ und 50 µl PBS enthaltend 30 µl H₂O₂ gefolgt in sechzigminütigem Abstand von 50 µl PBS enthaltend 750 Units Interleukin II (rIL-II) wurden +24, +72 und +168 Stunden nach der Blaseninduktion vorgenommen. Die intravesikalen Applikationen und die Zellauswertungen erfolgten doppelblind.

Die erhaltenen Ergebnisse sind in den beigefügten Figuren 3, 4, 5 und 6 dargestellt. In den Figuren bedeuten rIL-II = Interleukin II und Ox = Oxidans. Der aus den Figuren ersichtliche Effekt wird im folgenden diskutiert:
Es ist bekannt, daß nicht-proliferierende Lymphozyten in Kultur nur maximal 4 Tage überleben. Die allein mit rIL-II behandelte Blase wurde nach 120 Stunden geerntet. Die Lymphozyten überlebten nicht länger als 4 Tage. Die mit rIL-II + Oxidans behandelte Blase zeigte in Kultur gebracht ohne weitere in vitro Behandlung noch nach 15 Tagen in der Zellkulturflasche dichte Zellkonfluenz. Daraus folgt: Der nach 218 Stunden registrierte Zellanstieg der Lymphozyten nach intravesikaler Behandlung mit dem lymphozytenspezifischen Wachstumsfaktor rIL-II, potenziert durch die Behandlung mit H₂O₂, zeigt eine echte zellteilungsbedingte Zellvermehrung an. Das gleiche gilt auch für die Makrophagenvermehrung in der allein mit Oxidans behandelten Blase. Die Makrophagen wurden mit den klassischen Methoden (Fc-Rezeptor, IA-Oberflächenantigen, unspezifische Esterasereaktion positiv, Peroxidase-Reaktion negativ) identifiziert. Der Lymphozytenanstieg nach 96 und 120 Stunden in den mit rIL-II behandelten Blasen, deutlich potenziert durch das H₂O₂, ist hingegen nur auf eine Zellvermehrung durch vermehrte Einwanderung zurückzuführen. Aus diesem Grund überleben die Lymphozyten lediglich 4 Tage in vitro.

### Beispiel 2

Mit Hilfe der zytofluorometrischen Analyse von Blasenleukozyten wird gezeigt, daß die Kombination von comitogenem Oxidans mit Interleukin II, die zu einer Potenzierung der Interleukin II-Wirkung führt, die Expression des HLADR-Komplexes auf den T-Helferzellen deutlich vermehrt. Der Versuchsansatz war folgender:
Am Tag 0 wurden auf der volaren Seite beider Unterarme 4 intraepidermale Blasen mit Cantharidin induziert. 24 Stunden später wurden die Blasen folgendermaßen behandelt:
- Blase 1: (Kontrollblase) 50 µl physiologische Kochsalzlösung intravesikal
- Blase 2: Interleukin II 1000 Einheiten
- Blase 3: 50 µl 6,4 µmol H₂O₂ in PBS
- Blase 4: ebenfalls 50 µl H₂O₂ 6,4 µmol in PBS

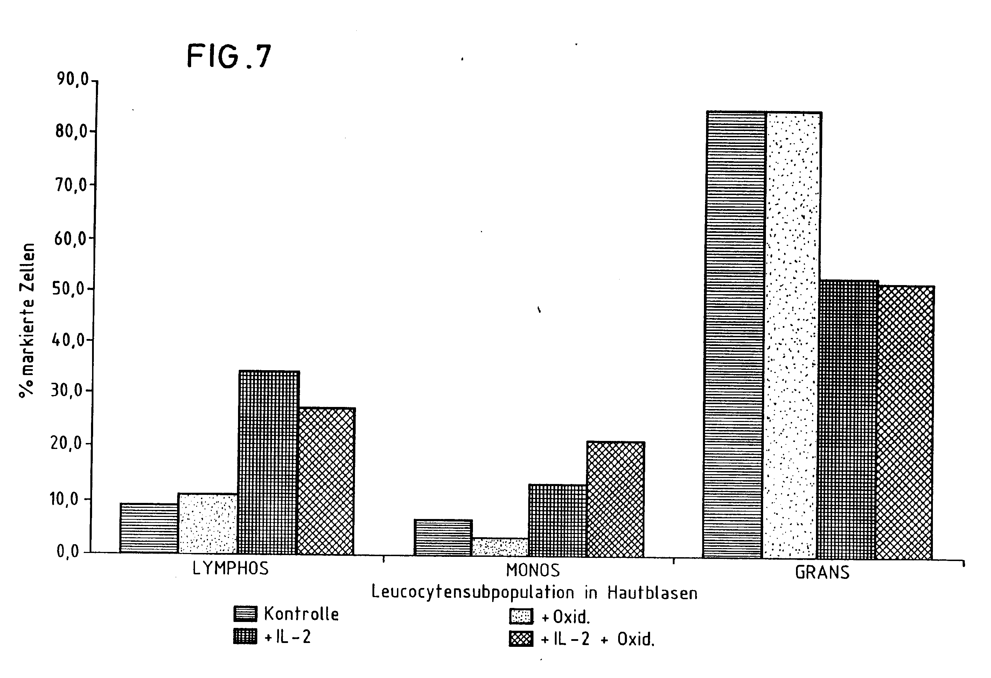

Eine Stunde später wurde in die Blase 4 die gleiche Menge von Interleukin II injiziert wie in die Blase 2. 80 Stunden nach der Blaseninduktion wurden alle Blasen durch vollständige Entnahme geerntet und nach Markierung mit monoklonalen fluoreszenzmarkierten Antikörpern cytofluorometrisch analysiert. Mitogene Effekte waren nach diesem kurzen Zeitintervall, d.h. 48 Stunden nach Injektion von Interleukin II, noch nicht zu erwarten. Dies wird durch die Ergebnisse, welche in den Figuren 7 bis 10 wiedergegeben sind, bestätigt.

Zur Markierung und Charakterisierung der Leukozyten wurden als monoklonale Antikörper verwendet: Anti-Leu 2a, 3a, 4, 5, 7, 11a, 11b, 12 und M3, Anti-Leucocyte (HLE-1), Anti-IL2-Rezeptor und Anti-HLA-DR, sämtlich im Handel erhältlich (Becton Dickinson).

Die Expression von Klasse II Antigenen (HLA-DR) auf T-Helferzellen, die Anwesenheit von zalreichen und mit hoher Dichte HLA-DR exprimierenden akzessorischen Zellen weist auf das hohe Immunisierungspotential der intraepidermalen Leukozyten hin.

## Patentansprüche

1. Verfahren zur Erzeugung syngener Zellen (Zellen mit identischer Erbanlage) mit hohem Immunisierungspotential und hoher Lebensdauer in vivo in einer Blase zwischen Dermis und Epidermis oder innerhalb der Epidermis,
**dadurch gekennzeichnet,** daß man eine Vermehrung und Konditionierung der Zellen durch Einführen eines oder mehrerer zellspezifischer Wachstumsfaktoren (Peptide und Proteine), die durch rezeptorvermittelte Endozytose aufgenommen werden und die Zellteilung (Mitose) und Differenzierung fördern) nicht toxischer Konzentrationen von einem oder mehreren Oxidationsmitteln, deren Redoxpotential dem des Peroxidase-Wasserstoffperoxid -Komplexes entspricht oder einer Kombination beider, in die Blase bewirkt und daß die Zellen in steriler Weise geerntet werden.

2. Verfahren nach Anspruch 1,
**dadurch gekennzeichnet,** daß zunächst das Oxidationsmittel eingeführt wird und dessen Wirkung anschließend durch Einführen der Wachstumsfaktoren potenziert wird.

3. Verfahren nach einem der Ansprüche 1 oder 2,
**dadurch gekennzeichnet,** daß die Wachstumsfaktoren und/oder nicht toxische mikromolare Konzentrationen der Oxidationsmittel wiederholt eingeführt werden.

4. Verfahren nach einem der vorhergehenden Ansprüche,
**dadurch gekennzeichnet,** daß die Blasenflüssigkeit nach der Induktion der Blase teilweise oder vollständig durch Donor-Serum ersetzt wird, das zu 10 bis 90 % mit einem sterilen Zellkulturmedium verdünnt und mit Zellen anderer Körperteile versetzt ist.

5. Verwendung der gemäß dem Verfahren eines der Ansprüche 1 bis 4 erhaltenen Zellen zur Herstellung von Arzneimitteln.

6. Verwendung der gemäß einem der Verfahren der Ansprüche 1 bis 4 erhaltenen Zellen zur Herstellung grober Mengen von Biomolekülen, wie insbesondere monoklonalen Antikörpern in vitro.

7. Verwendung der gemäß dem Verfahren eines der Ansprüche 1 bis 4 erhaltenen konditionierten Zellen als zytotoxische T-Zellen und/oder NK-Zellen gegen körpereigene oder -fremde Tumorzellen in vivo.

8. Verwendung der gemäß dem Verfahren eines der Ansprüche 1 bis 4 erhaltenen konditionierten Zellen zur Herstellung antiidiotypischer Antikörper und T-Zell-Klone in vivo.

9. Arzneimittel,
**dadurch gekennzeichnet,** daß es nach dem Verfahren der Ansprüche 1 bis 4 hergestellte syngene Zellen (Zellen mit identischer Erbanlage) mit hohem Immunisierungspotential und hoher Lebensdauer enthält.

## Claims

1. Method for the in vivo production of syngeneic cells (cells of an identical genetic origin) with a high immunisation potential and a high survival rate in a blister between dermis and epidermis or within the epidermis characterized in that a multiplication and conditioning of cells is achieved by introducing into the blister either one or several cell-specific growth factors (peptides and proteins), which are taken up by receptor-mediated endocytosis and which promote cell division (mitosis) and cell differentiation, or non-toxic concentrations of one or several oxidants, the redox potential of which corresponds to that of the peroxidase - hydrogen superoxide complex, or a combination of both and that these cells are harvested in a sterile manner.

2. Method according to claim 1 characterized in that the oxidant is introduced first and in that its effect is subsequently potentiated by the introduction of the growth factors.

3. Method according to one of the claims 1 or 2 characterized in that the growth factors and/or non-toxic micromolar concentrations of the oxidant are introduced repeatedly.

4. Method according to any of the previous claims characterized in that the blister liquid is partially or completely replaced by donor's serum, which is 10 to 90 % diluted with a sterile cell culture medium and supplemented with cells of other parts of the body.

5. Use of the cells obtained according to the method of any of the claims 1 to 4 for the production of therapeuticals.

6. Use of the cells obtained according to the method of any of the claims 1 to 4 for the in vitro production of substantial amounts of biomolecules, especially such as monoclonal antibodies.

7. Use of conditioned cells obtained according to the method of any of the claims 1 to 4 as cytotoxic T-cells and/or NK-cells against the body's own tumour cells or tumour cells from a different individual, in vivo.

8. Use of conditioned cells obtained according to the method of any of the claims 1 to 4 for the production of anti-idiotypic antibodies and T-cell clones, in vivo.

9. Therapeutical characterized in that it contains syngeneic cells (cells of identical genetic descendance) obtained according to the method of any of the claims 1 to 4 having a high immunisation potential and high survival rate.

## Revendications

1. Méthode pour la production in vivo de cellules syngéniques (cellules d'origine génétique identique) avec un potentiel d'immunisation élévé et une grande durée de survie dans une bule entre la derme et l'épiderme ou à l'intérieur de l'épiderme characterisée en ce que l'on obtient une multiplication et conditionnement des cellules par l'introduction soit d'un ou plusieurs facteurs de croissance spécifiques pour certains types de cellules (peptide ou protéine), qui sont accueillis par endocytose de médiateur des récepteurs et qui promotent la division cellulaire (mitose) et la différentiation cellulaire, soit de concentrations non-toxiques d'oxidants, dont le potentiel redox correspond à celui du complexe peroxidase - hydrogène superoxide ou d'une combinaison des deux dans la bule et en ce que ces cellules sont récoltées d'une manière stérile.

2. Méthode selon la revendication 1 characterisée en ce que l'oxidant est introduit d'abord et que son action est potentialisée par l'introduction de facteurs de croissance par la suite.

3. Méthode selon une des revendications 1 ou 2 characterisée en ce que les facteurs de croissance et/ou des concentrations micromolaires non-toxiques sont introduits à plusieurs reprises.

4. Méthode selon une des revendications spécifiées ci-dessus characterisée en ce que le liquide de la bule est remplacé partiellement ou complêtement par le sérum du donneur dilué de 10 à 90 % par un milieu de culture cellulaire stérile auquel des cellules d'autres parties du corps ont été rajoutées.

5. Utilisation in vitro des cellules obtenues selon la méthode des revendications 1 à 4 pour la production de remèdes.

6. Utilisation des cellules obtenues selon la méthode des revendications 1 à 4 pour la production de quantités importantes de biomolecules, telles que notamment des anticorps monoclonaux.

7. Utilisation in vivo des cellules conditionnées obtenues selon la méthode des revendications 1 à 4 comme cellules T cytotoxiques et/ou cellules NK contre des cellules tumorales du même ou d'un autre individu.

8. Utilisation in vivo des cellules conditionnées obtenues selon la méthode des revendications 1 à 4 pour la production des anticorps anti-idiotypiques et des clones de cellules T.

9. Médicament, characterisé en ce qu'il contient des cellules syngéniques (cellules d'origine génétique identique) avec un potentiel d'immunisation élévé et une grande durée de survie, obtenues selon la méthode des revendications 1 à 4.
